Europäisches Patentamt

(19) European Patent Office

Office européen des brevets

(11) Numéro de publication: **0 067 487**
**A1**

(12) # DEMANDE DE BREVET EUROPEEN

(21) Numéro de dépôt: **82200712.6**

(22) Date de dépôt: **10.06.82**

(51) Int. Cl.³: **A 61 B 6/02**
**A 61 B 6/00**

(30) Priorité: **12.06.81 FR 8111621**

(43) Date de publication de la demande:
**22.12.82 Bulletin 82/51**

(84) Etats contractants désignés:
**BE DE FR GB IT**

(71) Demandeur: **S.A. Massiot-Philips Matériel Médical**
**177, rue de Bezons**
**F-78420 Carrières sur Seine(FR)**

(84) Etats contractants désignés:
**FR**

(71) Demandeur: **N.V. Philips' Gloeilampenfabrieken**
**Groenewoudseweg 1**
**NL-5621 BA Eindhoven(NL)**

(84) Etats contractants désignés:
**BE DE GB IT**

(72) Inventeur: **Louiday, André**
**Société Civile S.P.I.D. 209, rue de l'Université**
**F-75007 Paris(FR)**

(74) Mandataire: **Souquet, Georges et al,**
**Société Civile S.P.I.D. 209, rue de l'Université**
**F-75007 Paris(FR)**

(54) Table radiologique pour incidence et focale variables et planigraphie.

(57) L'invention concerne une table de radiologie dans laquelle la colonne (4, 5) portant la source de rayons X (7) est télescopique et inclinable par rapport au plan (1) sur lequel se trouve le patient. Dans la table conforme à l'invention, l'inclinaison et les variations de la longueur de la colonne sont générées par un seul moteur (11) porté par la base (4) de la colonne. L'arbre de sortie dudit moteur portant une manivelle 9 dont le maneton 12 suit simultanément d'une part un guide 3 fixé au bâti de table, d'autre part un guide 6 lié à la partie télescopique 5 de la colonne.
Application : Radiographie médicale.

FIG.3

EP 0 067 487 A1

1

# TABLE RADIOLOGIQUE POUR INCIDENCE ET FOCALE VARIABLES ET PLANIGRAPHIE

L'invention concerne une table radiologique comportant un bâti fixe ou mobile supportant un plan sur lequel repose le patient, une colonne portant la source de rayons X, un support de récepteur d'image, la colonne étant composée d'une base tubulaire de longueur constante, articulée sur le bâti autour d'un axe d'inclinaison qui est perpendiculaire à la direction longitudinale de la colonne, et d'une partie télescopique portant la source et susceptible de coulisser à l'intérieur de la base tubulaire, l'inclinaison de la colonne étant réglable, et sa longueur entre l'axe d'inclinaison et la source étant d'une part réglable à plusieurs valeurs, indépendantes de l'inclinaison ou d'autre part asservie à ladite inclinaison pour l'obtention d'une trajectoire de source rectiligne de balayage tomographique. Une telle table est connue du brevet français n° 1 569 601. Elle est utilisée, en radiologie médicale, tout d'abord pour des prises de vues obliques, dites "incidences", la source de rayons X et le récepteur (film photographique ou amplificateur de luminance) étant fixes mais alignés sur un axe non perpendiculaire au plan sur lequel est placé le patient. Ce réglage est réalisé par un premier moteur qui incline la colonne. La table décrite dans le brevet cité permet également de réaliser des clichés avec plusieurs distances source-patient (dites "focales"), cela par mise en oeuvre d'un second moteur, un verrin en fait, agissant sur la longueur de la colonne télescopique. Enfin, cette table permet la réalisation de radiographies tomographiques par combinaison de l'inclinaison et de l'allongement de la colonne télescopique, la linéarité du déplacement de la source étant obtenue par un profil de came adéquat, solution qui était déjà celle exposée par le brevet allemand n° 1 118 928.

La table selon le brevet français 1 569 601 nécessite donc deux moteurs, montés sur la colonne, ce qui est lourd et coûteux. La présente invention se caractérise par le fait qu'inclinaison et variations de longueur de la colonne sont générées par un seul moteur porté par la base tubulaire de la colonne, l'arbre de sortie dudit moteur, parallèle à l'axe d'inclinaison et distinct de ce dernier, portant une manivelle dont le maneton suit simultanément d'une part un guide fixé au bâti, pour l'inclinaison, et d'autre part un guide lié à la partie télescopique de la colonne, pour les variations de longueur. La combinaison des deux mouvements imposés par le moteur, via la manivelle et son maneton, d'une part à la base de la colonne elle-même qui les porte, d'autre part à la partie télescopique, fait décrire à la source une trajectoire fermée dont les différentes zones sont utilisables pour obtenir les résultats énumérés plus haut.

Un autre inconvénient de l'art antérieur cité plus haut est de nécessiter des cames aux profils géométriques difficiles à usiner. Dans un mode particulier de réalisation de l'invention, le guide de variation de longueur est une glissière rectiligne perpendiculaire à la direction longitudinale de la colonne, l'allongement maximum de la colonne étant égal au double du rayon de la manivelle. Dans un autre mode de réalisation, sur la base tubulaire de la colonne, l'axe de la manivelle est situé à l'opposé de la source de rayons X par rapport à l'axe d'inclinaison et le guide d'inclinaison sur le bâti est une glissière rectiligne normale au plan du patient et dont la direction coupe l'axe d'inclinaison, le bras de levier b séparant les axes d'inclinaison et de manivelle étant supérieur au rayon m de la manivelle. La réalisation d'une table radiologique combinant ces deux modes de réalisation ne demande que des usinages rectilignes pour les guides utilisés. Dans une telle table, on appelle :

. i    l'angle d'incidence de la colonne par rapport à la normale au plan du patient.

. j    l'angle que fait la manivelle avec sa position parallèle à la colonne pour laquelle l'allongement est maximal.

. c    la longueur de la partie télécospique coulissante de la colonne.

. b    la longueur du bras de levier séparant les axes d'inclinaison et de manivelle.

. m    le rayon de la manivelle.

. R    la longueur utile de la colonne entre l'axe d'inclinaison à la source de rayons X.

Les coordonnées polaires de la trajectoire de la source dans le plan d'oscillation de la colonne, autour de la trace de l'axe d'inclinaison, sont R et i, liées par les expressions mathématiques :

$$R = c - b + m.\cos j \qquad et \qquad \frac{m}{\sin i} = \frac{b}{\sin (i + j)}$$

qui peuvent se résoudre, en éliminant le paramètre j, en :

$$R = c - b.\cos^2 i - e.\cos i . \sqrt{m^2 - b^2 \sin^2 i} \qquad avec \qquad e = \pm 1$$

soit, par développement (Mac LAURIN) :

$$R = c - (1 + e) .b.\cos^2 i - e. (m - b) - e. \frac{(m-b)^2}{2b} + e.\frac{(m^2-b^2)^2}{8b^3.\cos^2 i} \ ....$$

L'incidence maximum atteinte par la colonne est $i_{max} = arc \sin \frac{m}{b}$.

Lorsque la manivelle fait avec la colonne un angle j compris entre 0 et + 90° ou + 270° et + 360° (ou plus simplement entre - 90° et + 90°), la source de rayons X décrit une branche "haute" de trajectoire (cosj positif, e = - 1). Lorsque la manivelle fait avec la colonne un angle compris entre + 90° et + 270°, la source décrit une branche "basse" de trajectoire (cosj négatif, e = + 1).

4     **0067487**

La branche haute de trajectoire (e = - 1) a un "rayon" de colonne $R_h$ qui peut s'exprimer :

$$R_h = c + m - b + \frac{(m - b)^2}{2b} - \frac{(m^2 - b^2)^2}{8b^3.\cos^2 i} \quad \dots$$

Cette branche haute est assimilable à un arc de cercle de rayon (c + m - b), à condition que le rayon de la manivelle m soit peu différent de la longueur b du bras de levier, de l'ordre de (0,6.b) ou plus.

La branche basse de trajectoire (e = + 1) a un "rayon" de colonne $R_b$ qui peut s'exprimer :

$$R_b = c - m + b - 2b.\cos^2 i - \frac{(m - b)^2}{2b} + \frac{(m^2 - b^2)^2}{8b^3.\cos^2 i} \dots$$

Aux conditions posées précédemment (0,6.b < m < b) cette expression est assimilable à $R_b = c - m - b + 2b.\sin^2 i$, à rapprocher de l'équation d'une droite parallèle au plan du patient $R_d = \frac{c - m - b}{\cos i}$

A incidences faibles ( $|i| < 25°$), la branche basse de la trajectoire de source est assimilable à cette droite, à mieux que 1% près, à la condition que la hauteur c de la partie coulissante de la colonne soit approximativement c $\#$ 5.b + m.

La description qui suit et les dessins illustrent un exemple de réalisation d'une table radiologique conforme à l'invention.

La figure 1 est une représentation géométrique simplifiée d'une table conforme à l'invention et de sa colonne porte-source.

La figure 2 est une vue en perspective très schématique de la table symbolisée sur la figure 1, la colonne étant verticale et la source en position basse.

La figure 3 montre la colonne de la table précédente inclinée, la source en position haute.

La figure 4 est une vue en perspective des organes permettant au maneton de manivelle de la colonne des figures 2, 3, de suivre simultanément deux glissières.

La figure 5 est une coupe longitudinale de la partie basse de la colonne.

La figure 6 est une coupe transversale de la colonne. Sur le schéma de la figure 1, des parties du bâti de la table sont représentées avec des hachures attenantes, c'est-à-dire le plan 1 sur lequel repose le patient à radiographier, représenté horizontal, l'articulation 2 de la colonne d'axe parallèle au plan 1, appelé axe d'inclinaison de la colonne et désigné également par la référence 2, et une glissière rectiligne 3, perpendiculaire au plan 1, donc ici verticale, et à l'axe d'inclinaison 2, glissière participant à l'inclinaison de la colonne comme expliqué plus loin.

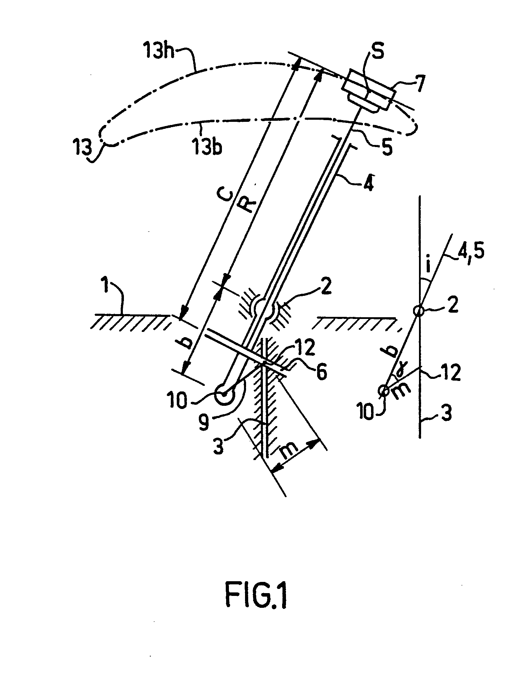

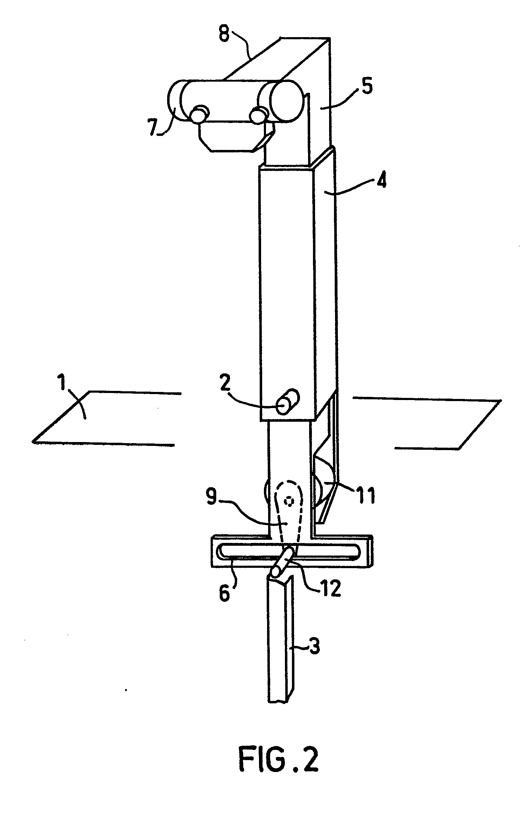

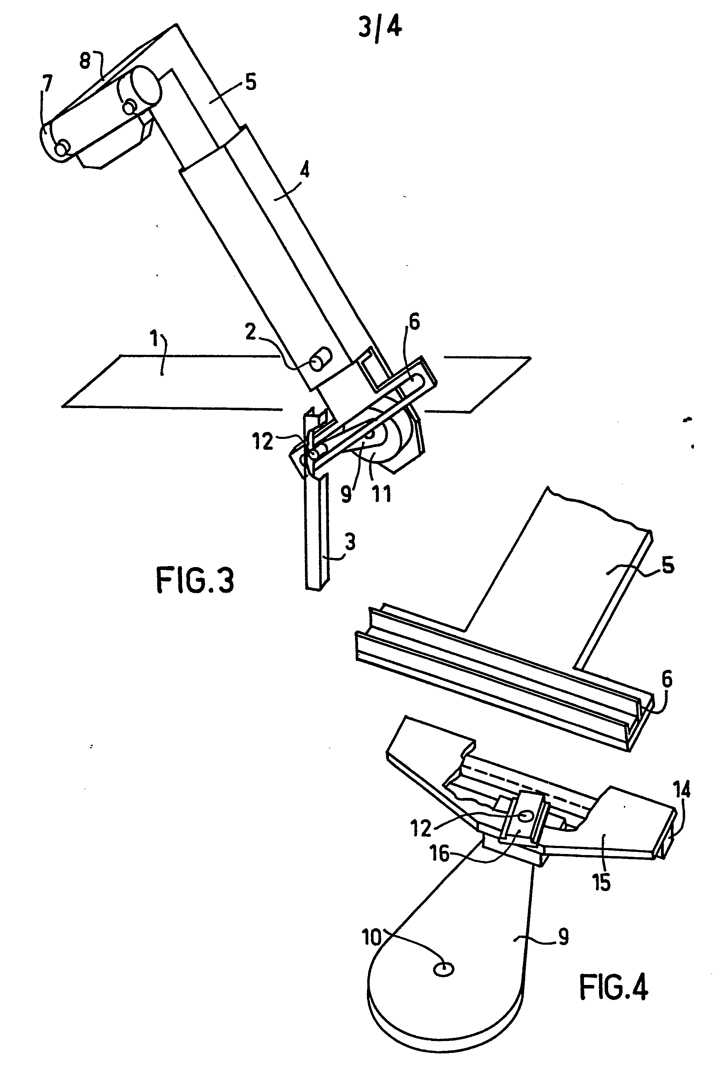

Autour de l'articulation 2 est montée à rotation la base tubulaire 4 d'une colonne télescopique supportant la source S de rayons X. A l'intérieur de cette base tubulaire 4 peut coulisser une partie télescopique 5 de la colonne au sommet de laquelle est fixée la source S et dont la partie inférieure, faisant saillie de la section fermée de la base tubulaire 4 (voir figures 2, 3) porte une glissière rectiligne 6, qui lui est perpendiculaire et se trouve dans le plan d'inclinaison de la colonne. La source S, considérée comme ponctuelle, est située dans un tube émetteur de rayons X, lui-même contenu dans une enveloppe 7 (appelée "gaine") de protection anti-rayons X. La gaine 7 est disposée à l'extrémité d'un bras 8 parallèle à l'articulation 2 et formant potence avec la colonne 4, 5. Le schéma géométrique de la figure 1 constitue donc la projection, parallèlement à l'articulation 2, dans le plan passant par la source S et perpendiculaire à cette articulation (plan d'inclinaison de la source) des organes générant le mouvement de la source.

A l'extrémité basse de la colonne sur la base tubulaire 4 est montée à rotation une manivelle 9 autour d'un axe 10

parallèle à l'axe d'inclinaison 2. La manivelle 9 est animée par un moteur 11, schématisé sur les figures 2 et 3, qui est fixé à la base tubulaire 4 de la colonne. La masse de ce moteur permet d'équilibrer partiellement la colonne dont la partie télescopique porte la gaine 7 de masse d'une trentaine de kilogrammes. A l'extrémité libre de la manivelle 9 est monté un maneton 12 représenté de façon simplifiée par un tronçon cylindrique sur les figures 2 et 3. Le maneton 12 est engagé simultanément dans les deux guides rectilignes 3 et 6.

La rotation de la manivelle 12 autour de son axe 10 entraîne d'une part l'oscillation de la colonne 4, 5 autour de son axe d'inclinaison 2, d'autre part le va-et-vient de la partie coulissante 5 de la colonne dans sa base tubulaire 4. La conjugaison de ces deux mouvements fait décrire à la source S une trajectoire fermée 13 en forme de "banane" représentée par un trait mixte sur la figure 1.·

L'explication mathématique mentionnée précédemment s'applique à l'exemple de réalisation décrit ici. A la figure 1, déjà très schématique, est associé un dessin simplifié à même échelle montrant plus clairement le triangle (2, 12, 10) dont deux angles i et j et deux côtés b et m sont des paramètres de détermination de la trajectoire 13. La table radiologique décrite a été réalisée avec les dimensions suivantes : c = 1500 mm, b = 275 mm, m = 200 mm. Dans ces conditions, l'incidence maximale de la colonne est ± 46,6°. Lorsque la manivelle fait avec la colonne un angle j = 0, la partie coulissante 5 de la colonne est extraite au maximum, la source occupe le sommet de la branche haute (13h) de sa trajectoire. Le "rayon" de colonne est alors $R_h$ = 1425 mm. Cette branche 13 h est assimilable à un cercle de rayon $R_h$, à 1% près, sur environ ± 40° de part et d'autre de la normale au plan 1 du patient. La table permet donc de réaliser des clichés radiographiques avec toute incidence comprise dans cet intervalle.

Lorsque la manivelle 9 fait avec la colonne 4, 5 un angle de 180°, la colonne est rétractée au maximum, la source occupe le sommet de la branche basse (13b) de sa trajectoire. Le "rayon" de la colonne est alors $R_b$ = 1025 mm. Cette branche 13b est assimilable à un segment de droite, parallèle au plan 1 du patient, à 1% près, sur environ ± 27° de part et d'autre de la normale au plan 1. La table permet donc un balayage tomographique rectiligne sur cet intervalle.

Enfin, la colonne étant normale au plan 1, pour passer de la "focale" 1025 mm à la "focale" 1425 mm et inversement, il suffit de faire tourner la manivelle de 180°.

Les figures 4 à 6 illustrent de façon plus concrète certains détails de réalisation de la table dont le fonctionnement a été expliqué précédemment. La figure 4 montre la manivelle 9 montée sur son axe moteur 10 et portant à son extrémité le maneton 12. Celui-ci ne frotte pas directement dans les glissières rectilignes d'inclinaison et d'allongement de colonne mais sert de pivot à deux organes plus aptes à effectuer ce glissement. La glissière 6 est constituée par un profilé métallique en U, fixé à la base de la partie coulissante 5 de la colonne perpendiculairement à la colonne, l'ouverture du U étant latérale par rapport à la colonne. Dans ce profilé glisse un rail 14 de section correspondante, par exemple rectangulaire, porté par un flasque 15 articulé sur le maneton 12. Cette coulisse en deux parties (dont l'implantation peut être inversée) permet de réduire la longueur totale de la glissière à la base de la colonne (dans le schéma des figures 2 et 3, la longueur de glissière serait de 2 x m = 400 mm au moins). L'association d'une glissière en acier phosphaté et d'un rail en bronze, par exemple, permet un bon glissement. La glissière 3, destinée à l'inclinaison de la colonne, est de section rectangulaire et reçoit un curseur 16, en bronze également par exemple, qui est lui aussi articulé sur le maneton-pivot 12. Le curseur 16 et le flasque 15 sont montés par des roulements à aiguilles sur le pivot 12.

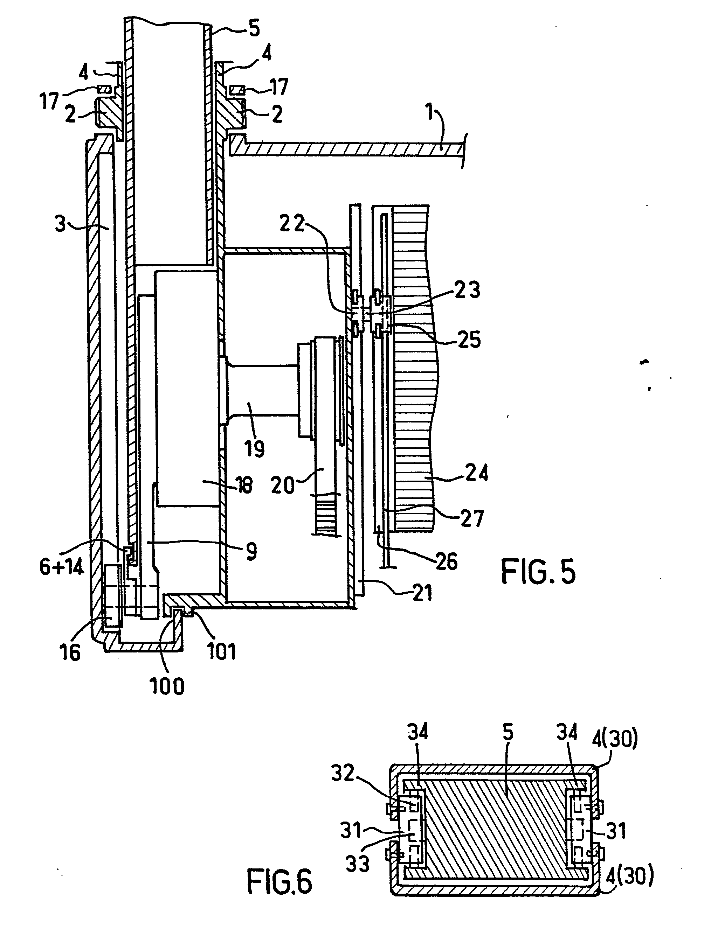

La figure 5 montre en coupe longitudinale partielle le bas de la colonne. La base tubulaire 4 porte deux tourillons 2 définissant son axe d'inclinaison, qui reposent dans des paliers à roulements solidaires du bâti de la table et du plan 1 (roulements non dessinés). Pour améliorer la stabilité du plan d'inclinaison de la colonne, un rail de guidage circulaire 100,
solidaire du bâti de la table, est suivi par un cavalier 101 fixé
à une structure liée à la base tubulaire 4 de la colonne, pendant
les mouvements de la colonne.

La manivelle 9 est montée sur l'arbre de sortie d'un
réducteur 18 qui peut être du type à couronne dentée déformable
(grand rapport de réduction). L'arbre d'entrée 19 de ce réducteur
est entraîné, via une poulie, par une courroie dentée 20 mise en
rotation par un moteur fixé à la base tubulaire 4 de la colonne.
Ledit moteur n'est pas dessiné sur la figure car il est décalé
par rapport à l'axe de colonne pour des raisons d'encombrement.
La structure supportant moteur et réducteur reçoit, sur une face
verticale parallèle au plan d'inclinaison, une glissière 21,
parallèle à la colonne, dans laquelle peut circuler un chariot 22
pivotant sur un tourillon 23 appartenant au support de récepteur .
d'image radiographique 24 (appelé "sériographe"). Le tourillon 23
est lui-même monté sur un curseur 25 dont la hauteur sur le
"sériographe" 24 est ajustable, sur un chemin 26, au moyen par
exemple d'une tige filetée 27 parallèle audit chemin et vissée
dans un taraudage du curseur 25. Le sériographe 24 est, par rapport
au bâti de table, monté sur des rails parallèles au plan d'inclinaison de la colonne et au plan du patient 1, de façon à pouvoir
se déplacer de ± 170 mm par rapport à sa position médiane (colonne
verticale). L'accouplement entre colonne et sériographe, réalisé
par le tourillon 23, permet d'asservir la position du récepteur
d'image (film ou amplificateur de luminance) à l'inclinaison de
la colonne. Par réglage de la hauteur du tourillon 23 (via la vis
27), il est possible, de façon classique, d'ajuster la hauteur de
"coupe" d'une radiographie tomographique.

La figure 6 est une coupe transversale de la colonne
4, 5. La base tubulaire 4 est constituée de deux fers en U (30)
en regard associés par des pièces de liaison internes 31 parallélépipédiques, sur trois faces desquelles sont montés des galets à
roulement 32, 33. La partie coulissante 5 de la colonne, dessinée
pleine pour simplifier, comporte sur deux faces des profils en U ouverts sur les pièces de liaison 31 de façon à coopérer avec les galets 32, 33 qui assurent le positionnement et le coulissement de la partie S dans la base 4.

REVENDICATIONS :

1. Table radiologique comportant un bâti fixe ou mobile supportant un plan (1) sur lequel repose le patient, une colonne (4, 5) portant la source (S) de rayons X, un support de récepteur d'image, la colonne étant composée d'une base tubulaire (4) de longueur constante, articulée sur le bâti autour d'un axe d'inclinaison (2) qui est perpendiculaire à la direction longitudinale de la colonne, et d'une partie télescopique (5) portant la source S et susceptible de coulisser à l'intérieur de la base tubulaire, l'inclinaison de la colonne étant réglable, et sa longueur entre l'axe d'inclinaison (2) et la source (S) étant d'une part réglable à plusieurs valeurs, indépendantes de l'inclinaison, ou d'autre part asservie à ladite inclinaison pour l'obtention d'une trajectoire de source rectiligne de balayage tomographique, caractérisée par le fait qu'inclinaison et variations de longueur. de la colonne (4, 5) sont générées par un seul moteur (11) porté par la base tubulaire (4) de la colonne, l'arbre de sortie dudit moteur, parallèle à l'axe (2) d'inclinaison et distinct de ce dernier, portant une manivelle (9) dont le maneton (12) suit simultanément d'une part un guide (3) fixé au bâti, pour l'inclinaison, et d'autre part un guide (6) lié à la partie télescopique (5) de la colonne, pour les variations de longueur.

2. Table selon la revendication 1, caractérisée par le fait que le guide (6) de variation de longueur est une glissière rectiligne perpendiculaire à la direction longitudinale de la colonne, l'allongement maximum de la colonne étant égal au double du rayon de la manivelle (9).

3. Table selon l'une quelconque des revendications 1 ou 2, caractérisée par le fait que sur la base tubulaire (4) de la colonne, l'axe de la manivelle (9) est situé à l'opposé de la source S de rayons X par rapport à l'axe d'inclinaison (2) et le guide d'inclinaison (3) sur le bâti, est une glissière rectiligne normale au plan du patient (1) et dont la direction coupe l'axe d'inclinaison, le bras de levier b séparant les axes d'inclinaison (2) et de manivelle (9) étant supérieur au rayon m de la manivelle (9).

4. Table selon les revendications 2 et 3 prises ensemble, caractérisée par le fait que l'écart entre le rayon de la manivelle m et la longueur b du bras de levier est de l'ordre de 40% ou moins et que la longueur de la partie coulissante de la colonne entre la glissière d'allongement et la source est voisine de c = 5.b + m.

FIG.1

0067487

FIG.2

FIG.3

FIG.4

FIG.5

FIG.6

0067487

**Office européen des brevets**

## RAPPORT DE RECHERCHE EUROPEENNE

Numéro de la demande

EP  82 20 0712

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-A-2 352 531 (COMPAGNIE GENERALE DE RADIOLOGIE) * page 4, ligne 24 - page 8, ligne 31; figures 1-3 * | 1-3 | A 61 B    6/02 A 61 B    6/00 |
| | --- | | |
| D,A | FR-A-1 569 601 (ETS. DUTERTRE & CIE.) * page 3, colonne de gauche, ligne 26 - page 4, colonne de gauche, ligne 60; figures 1-6 * | 1,3 | |
| | --- | | |
| D,A | DE-B-1 118 928 (SIEMENS-REINIGERWERKE AG) * colonne 2, ligne 20 - colonne 3, ligne 64; figures 1-3 * | 1 | |
| | --- | | |
| A | FR-A-1 365 686 (SIEMENS-REINIGERWERKE AG) * page 2, colonne de gauche, lignes 15-42; figure 1 * | 1,3 | **DOMAINES TECHNIQUES RECHERCHES (Int. Cl. ³)** |
| | --- | | |
| A | DE-A-2 120 344 (G. ROSSI ET B. D'APUZZO) * page 3, ligne 17 - page 4, ligne 18; figure 1 * | 1,3,4 | A 61 B    6/02 A 61 B    6/00 |
| | ----- | | |

Le présent rapport de recherche a été établi pour toutes les revendications

| Lieu de la recherche | Date d'achèvement de la recherche | Examinateur |
|---|---|---|
| LA HAYE | 15-09-1982 | ZILLIOX J.M. |